Europäisches Patentamt

European Patent Office

Office européen des brevets

(11) Publication number: **0 112 104**
**B1**

# EUROPEAN PATENT SPECIFICATION

(45) Date of publication of patent specification: **18.05.88**

(51) Int. Cl.⁴: **A 61 M 1/28**

(21) Application number: **83307358.8**

(22) Date of filing: **02.12.83**

(54) **Apparatus for peritoneal dialysis.**

(30) Priority: **10.12.82 JP 215497/82**

(43) Date of publication of application:
**27.06.84 Bulletin 84/26**

(45) Publication of the grant of the patent:
**18.05.88 Bulletin 88/20**

(84) Designated Contracting States:
**DE FR GB SE**

(56) References cited:
**EP-A-0 028 371**
**EP-A-0 097 432**
**DE-A-2 734 075**
**DE-B-2 755 214**
**FR-A-2 035 926**
**GB-A-2 052 303**

(73) Proprietor: **Japan Medical Supply Co. Ltd.**
**12-17 Kako-Machi,**
**Hiroshima, 733/Japan (JP)**

(72) Inventor: **Yamane, Seiji**
**50-2, Takatori Yasuhuruichi-cho**
**Asaminami-ku Hiroshima (JP)**
Inventor: **Oda, Kazuyuki**
**2220-2, Nagatsuka Gion-cho**
**Asaminami-ku Hiroshima (JP)**

(74) Representative: **Crampton, Keith John Allen et al**
**D YOUNG & CO 10 Staple Inn**
**London WC1V 7RD (GB)**

Courier Press, Leamington Spa, England.

## Description

This invention relates to apparatus for peritoneal dialysis. More particularly, this invention is concerned with automatic apparatus for peritoneal dialysis which provides an essentially closed controlled fluid circuit for the dialysate.

Peritoneal dialysis is a method for removing toxic materials from the blood using the peritoneum. Recently several attempts have been made to automate peritoneal dialysis by the use of a fluid circuit. When compared with an artificial kidney, this system is easier to operate and hence has gradually gained increased acceptance. However, conventional peritoneal dialysis systems often use a solution tank for storing dialysate, and the fluid circuits include a large number of circuit portions making up the circulation system, so that the quantity and quality of the dialysate cannot be managed very conveniently.

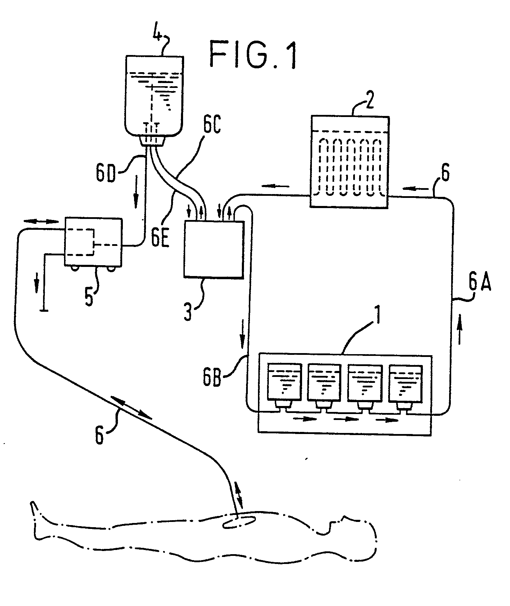

Figure 1 of the accompanying drawings illustrates a typical conventional automatic system as disclosed in Japanese Patent Publication No. JP—A—2679/1972. Referring to Figure 1, it can be seen that the system consists of a dialysate-feed unit 1, a constant-temperature tank 2, a dialysate transfer machine 3, a dialysate storage tank 4, a selector 5, and transfer tubing 6 connecting the members 1 to 5.

The dialysate feed unit 1 usually consists of a single large solution tank or two or more solution bottles connected in series. A transfer tube 6A transfers dialysate from the dialysate feed unit 1 to the constant-temperature tank 2, while a transfer tube 6B returns dialysate in excess of a predetermined injection quantity to the dialysate-feeding unit 1 from a circulation circuit. The constant-temperature tank 2 heats the dialysate to be injected into the patient to a temperature substantially equal to body temperature. Heating is effected by coiling the transfer tubing into a spiral and immersing it in a hot-water tank, or by passing hot air over the transfer tubing. The dialysate transfer machine 3 transfers a quantity of dialysate sufficient to be injected into the patient from the dialysate-feed unit 1 to the dialysate storage tank 4. A low-speed pump or the like is used as the transfer machine 3. The dialysate storage tank 4 stores the quantity of the dialysate for injection into the patient and consists of a container equipped with an inlet passage 6C, an outlet passage 6D, and a return passage 6E returning dialysate in excess of the predetermined quantity to the transfer machine 3. The desired predetermined quantity is decided by positioning a graduated scale on the wall and moving the end of the tube of the return passage 6E to an appropriate height. The selector 5 injects the dialysate sent from the dialysate storage tank 4 into the patient, and removes and discharges the dialysate from the patient after the passage of a predetermined period of time. For this reason, the transfer tubing 6 is formed into a T-shape within the selector 5. The first branch of the T-shaped tubing communicates with the dialysate storage tank 4, the second with the patient, and the third is for discharge. The transfer passage connected to the patient is used in common for both injection and discharge.

EP—A—0028371 describes a method and apparatus for automatic semi-continuous peritoneal dialysis wherein a predetermined amount of dialysate is pumped from a supply bag or bags to the peritoneal cavity of a patient and pumped back from the peritoneal cavity to the supply bag or bags for a predetermined number of cycles, the peritoneal cavity, pump and supply bag or bags forming a closed circuit, and the dialysate being maintained at a desired temperature with a heater.

GB—A—2052303 describes apparatus for both haemodialysis and peritoneal dialysis comprising a unit for measuring a patient's change in weight during treatment and generating a change-of-weight signal, means for controlling the differential pressure between the dialysate and the blood, and a unit for controlling the composition of the dialysate which is fed by pure water and dialysate concentrate, wherein the unit for measuring the patient's change of weight is connected to a unit for monitoring and controlling the dialysis treatment, the desired rate of change-of-weight being introduced into the monitoring unit as a set-point value and the monitoring and control unit being so arranged that with the aid of the change-of-weight signal it controls the mechanical and/or osmotic differential pressure acting across the dialysis diaphragm such that the actual change-of-weight conforms on average to the set point value.

EP—A—0097432 describes peritoneal dialysis apparatus, but is only relevant in accordance with Article 54, paragraphs (3) and (4) of the European Patents Convention. In particular EP—A—0097432 describes a dialysis apparatus which includes first means for supplying dialysis fluid, second means for receiving, heating and weighing dialysis fluid, third means for receiving and weighing a quantity of dialysis fluid, a catheter, and structure which supports the first means above the second means, the second means above the catheter, and the third means below the catheter. A first flow path interconnects the first and second means, while a second flow path interconnects the second means in the catheter. A third flow path interconnects the catheter in the third means. Valve means are provided for selectively (i) blocking the first and third paths while leaving the second path open, (ii) blocking the second and third paths while leaving the first path open, and (iii) blocking the second path while leaving the first and third paths open. A timer/computer is provided which is able to maintain mode (i) until a preselected reduction in weight occurs at the second means, following which it switches to and maintains mode (ii), following which it switches to and maintains mode (iii).

One problem with prior art apparatus is that the dialysate may frequently become contaminated. The possibility of contamination is greater for apparatus having a tank type dialysate-feed unit than for apparatus having a series of bottles for the dialysate feed unit, but even if the dialysate-feed unit comprises series of bottles, contamination is likely to increase whenever the dialysate is topped up. Moreover, when a circulation system is used to maintain the temperature, all the dialysate in the entire apparatus must be replaced at the same time in order to refresh the dialysate. Furthermore, prior art apparatus may include a number of portions which are exposed to the atmosphere, including the dialysate storage tank, and this may result in contamination problems. In addition, differences in osmotic pressure resulting from different components of the dialysate may have a strong influence on the condition of the patient; but in prior-art apparatus, it has been difficult to adjust the osmotic pressure in the dialysate-feed unit accurately, and to control, in practice, how much dialysate is injected into and discharged from the patient's body.

The present invention provides apparatus for peritoneal dialysis comprising a dialysate-feeding unit comprising a plurality of dialysate containers of the same type, a dialysate storage unit for temporarily storing dialysate removed from the dialysate-feed unit, means for transferring dialysate from the feed unit to the storage unit, a heater for heating the dialysate before it is injected into the patient's body, and a dialysate discharge unit for receiving the dialysate after it has been passed through the patient's body, all of the units being connected into a controllable fluid circuit by transfer tubing, the fluid circuit being controlled by weighing means for the dialysate storage unit and the dialysate discharge unit, which are electrically connected to a calculator to calculate the quantities of dialysate injected and discharged into and from the patient from the two numerical values obtained from the weighing means, and each dialysate container being removably affixed to the fluid circuit, characterised in that each dialysate container is connected in parallel to another and contains a dialysate of different composition to another such that the dialysate of each container can be admixed with the dialysate from another container at the inlet of the transfer tubing, to produce a composite dialysate having a desired osmotic pressure appropriate to the condition of the patient, and in that the calculator is connected to a display for displaying the calculated injected and discharged quantities.

Embodiments of the invention will be hereinafter described by way of non-limiting example with reference to the accompanying drawing, in which:—

Figure 1, as mentioned above, is a schematic circuit diagram of a typical prior-art automatic apparatus for peritoneal dialysis;

Figure 2 is a front view of an embodiment of an automatic apparatus for peritoneal dialysis in accordance with the present invention; and

Figure 3 is a schematic circuit diagram of the apparatus shown in Figure 2.

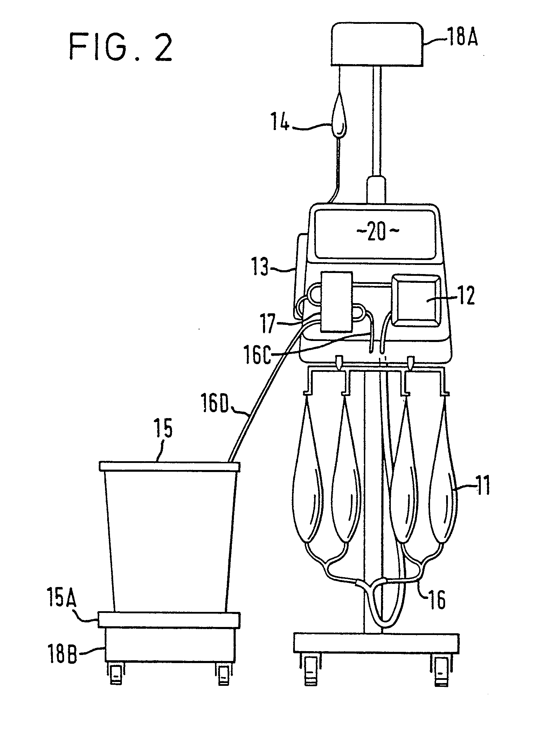

In Figure 2, apparatus for peritoneal dialysis includes dialysate bags 11, a dialysate-feed unit which, in this instance, is a pump 12 which pumps the dialysate from the dialysate bags 11, a heater 13, a dialysate storage bag 14 and a dialysate discharge meter 15A acting in conjunction with a dialysate discharge tank 15 to form a discharge unit. The members 11 to 15A are connected by transfer tubing 16 to form a fluid circuit. The apparatus also includes a transfer tubing switch 17 which changes the direction and branching of the circuit, and load cells 18A and 18B, which act as weighing means.

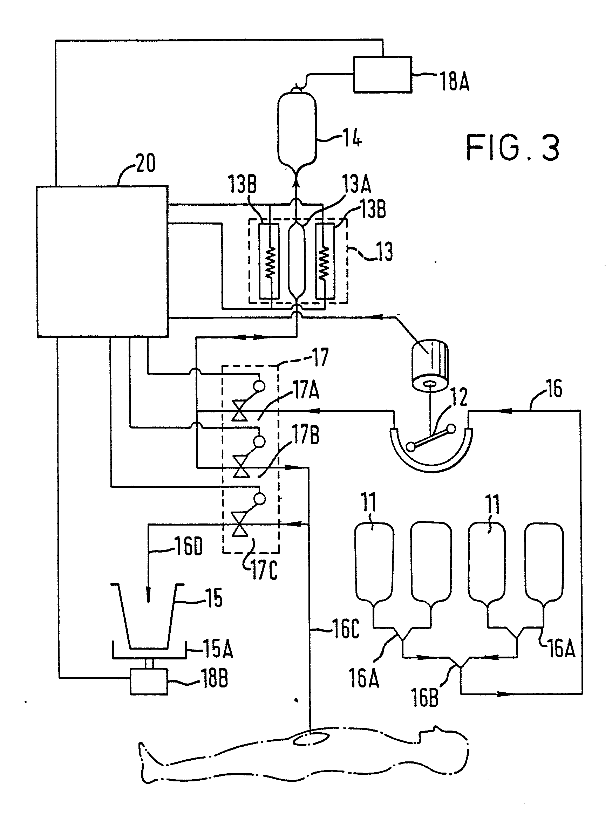

Figure 3 is a schematic circuit diagram of the construction of the apparatus described above, and is useful for explaining its function. In the present invention, the dialysate-feed unit consists of dialysate bags 11, which are of the same size and type, positioned at the same height, and connected in parallel with one another by confluent transfer tubing 16A and 16B. The dialysate is removed from all of these bags simultaneously by the pump 12.

In the apparatus of the invention, a number of dialysate bags containing dialysate of different composition are combined as shown in Figure 3, so that a composite dialysate having a desired osmotic pressure appropriate to the condition of the patient can be obtained by combining the several kinds of dialysates when mounting the dialysate bags 11. The osmotic pressure of the composite dialysate can be finely adjusted by replacing only one of the dialysate bags 11 in accordance with the condition of the patient. It is also possible to use resin or glass bottles instead of bags, but in this case, the interiors of the bottles must be kept at atmospheric pressure.

The composite dialysate prepared in the manner described above is pumped by pump 12 to the transfer tubing switch 17. This switch consists of a stricture device having clamps that constrict the transfer tubing, which is flexible at at least this part of the apparatus, to cut off the communication of the fluid or to establish open communication. It is equipped with a metering clamp 17A, an injection clamp 17B, and a discharge clamp 17C, and the branching and direction of the fluid circuit are altered by instructions from a controller 20. The dialysate, which is pumped through the tubing by pump 12, passes through the metering clamp 17A and is transferred to the dialysate storage bag 14. During this time, the injection clamp 17B is kept closed. When the metering clamp 17A opens, the weight of dialysate is measured by the load cell 18A using the dialysate storage bag 14 as a metering bag, and when the weight reaches a predesired weight set in advance in the controller 20, the pump 12 is stopped by an instruction from the controller. Next, while the metering clamp 17A and the discharge clamp 17C are kept closed, the injection clamp 17B is opened so that the dialysate in the storage bag 14 is injected into the patient's body through the transfer tubing 16C by the head of pressure. In the interim, the load cell 18A continues to monitor the weight of the dialysate storage bag 14, so that its change in weight can be used as a means of monitoring the weight of fluid injected.

Finally, the injection clamp 17B is closed and the discharge clamp 17C is opened while the metering clamp 17A is kept closed, the discharge passage for the dialysate is opened, so that the dialysate flowing from inside the patient's body flows back through the transfer tubing 16C, through discharge clamp 17C and through transfer tubing 16D into dialysate discharge tank 15. A dialysate discharge meter 15A acting in conjunction with load cell 18B weighs the discharged dialysate in the same manner as that of the load cell 18A. The quantities of dialysate injected and discharged can therefore be determined by calculating the difference between the injected weight and the discharged weight. The discharge operation can be effected simultaneously with the pumping and metering operation of the dialysate into the dialysate storage tank 14.

The heater 13 consists of a heating bag 13A of a synthetic plastics material through which the dialysate flows, and two flat electric heaters 13B, 13B' of a large thermal capacity. The heating bag is preferably partitioned so that the dialysate flows through it along a zig-zag path. The flat heaters are kept at a predetermined temperature by the controller 20, and sandwich the heating bag 13A between them so as to heat the dialysate by thermal conduction. Since the apparatus of the present invention does not generally use hot water or the like as the heating medium, as in some prior-art apparatus, the size of the dialyser as a whole can be made more compact and the control and use of the apparatus becomes much easier. Moreover, the use of this heater avoids the need for a circulation circuit for heating, and the dialysate can be kept at the optimum temperature by heating only the dialysate flowing through the heating bag 13A, both when the dialysate is being pumped into the dialysate storage unit, and when it is being injected into the human body.

The pump 12, the heater 13, and the transfer tubing switch 17 are all connected to the controller 20 by electric circuits, and the injections and discharge operations of the dialysate are under sequence control based on a timer incorporated within the controller 20. The controller 20, which is equipped with calculation means and display means, receives the values of the injections weight and discharged weight as monitored by the load cells 18A and 18B respectively, calculates the difference between those values and displays the difference on a real-time basis.

As described above, the automatic apparatus for peritoneal dialysis in accordance with the present invention enables a desired osmotic pressure of the dialysate to be selected by the combination of dialysates of differing osmotic pressures, which has not been previously possible in prior-art apparatus, and can also enable the quantity of dialysate injected into the patient to be delicately set in units of 10 grams. Moreover, temperature control can be easily effected. Since the apparatus, including the structure of the dialysate-feeding unit, the method of measuring the weight, and the position of the heating unit can all be arranged in a closed circuit, the number of connections in the transfer tubing is small, which makes the apparatus of the invention extremely hygienic.

The above described automatic apparatus of the present invention is easy for a doctor or nurse to handle, has a reduced likelihood of bacterial contamination, and can control the quality, quantity and temperature of the dialysate according to the condition of the patient.

**Claims**

1. Apparatus for peritoneal dialysis comprising a dialysate-feed unit comprising a plurality of dialysate containers (11) of the same type, a dialysate storage unit (14) for temporarily storing dialysate removed from the dialysate-feed unit, means (12) for transferring dialysate from the feed unit to the storage unit, a heater (13) for heating the dialysate before it is injected into a patient's body, and a dialysate discharge unit (15) for receiving the dialysate after it has been passed through the patient's body, all of the units being connected into a controllable fluid circuit by transfer tubing (16), the fluid circuit being controlled by weighing means (18A, 18B) for the dialysate storage unit (14) and the dialysate discharge unit (15), which are electrically connected to a calculator (20) to calculate the quantities of dialysate injected and discharged into and from the patient from the two numerical values obtained from the weighing means (18A, 18B), and each dialysate container (11) being removably affixed to the fluid circuit, characterised in that each dialysate container (11) is connected in parallel to another and contains a dialysate of different composition to another such that the dialysate of each container can be admixed with the dialysate from another container at the inlet of the transfer tubing (16) to produce a composite dialysate having a desired osmotic pressure appropriate to the condition of the patient, and in that the calculator (20) is connected to a display for displaying the calculated injected and discharged quantities.

2. Apparatus as claimed in claim 1 wherein the fluid circuit contains a transfer tubing switch (17) consisting of a metering clamp (17A), an injection clamp (17B), and a discharge clamp (17C), which co-operate to switch the flow of the dialysate from the storage unit (14) to the patient, or from the patient to the discharge unit (15).

3. Apparatus as claimed in either of claim 1 and 2 wherein the dialysate-feeding unit and/or the dialysate storage unit comprise bags (11, 14) suitable for holding dialysate.

4. Apparatus as claimed in any preceding claim wherein the heating unit comprises a synthetic resin bag (13A) provided at an intermediate portion of the fluid circuit, and heaters (13B) that sandwich the bag between them and that can heat the dialysate by thermal conduction.

5. Apparatus as claimed in any preceding claim wherein the heating unit (13) and the dialysate storage unit (14) are directly connected by a single length of transfer tubing.

## Patentansprüche

1. Vorrichtung zur peritonealen Dialyse mit einer Dialysatbeschickungseinheit mit mehreren Dialysatbehältern (11) gleichen Typs, einer Dialysatspeichereinheit (14) für zeitweilige Speicherung von Dialysat, das aus der Dialysatbeschickungseinheit entfernt wurde, Einrichtungen (12) zur Überführung von Dialysat von der Beschickungseinheit zu der Speichereinheit, einem Erhitzer (13) zum Erhitzen des Dialysats vor seinem Einspritzen in den Körper eines Patienten und einer Dialysatentnahmeeinheit (15) zur Aufnahme des Dialysats, nachdem es durch den Körper des Patienten gegangen ist, wobei alle Einheiten in einem steuerbaren Fließmittelkreis durch Überführungsleitungen (16) verbunden sind, der Fließmittelkreis durch Wiegeeinrichtungen (18A, 18B) für die Dialysatspeichereinheit (14) und die Dialysatentnahmeeinheit (15) gesteuert wird, die elektrisch mit einem Rechner (20) verbunden sind, um die in den Patienten eingespritzen und ihm entnommenen Dialysatmengen aus den beiden numerischen Werten zu berechnen, die man von den Wiegeeinrichtungen (18A, 18B) erhält, und jeder Dialysatbehälter (11) entfernbar in dem Fließmittelkreis befestigt ist, dadurch gekennzeichnet, daß jeder Dialysatbehälter (11) parallel zu einem anderen angebunden ist und ein Dialysat anderer Zusammensetzung als der andere enthält, so daß das Dialysat eines jeden Behälters mit dem Dialysat aus einem anderen Behälter am Einlaß der Überführungsleitung (16) vermischt werden kann, um ein zusammengesetztes Dialysat zu erzeugen, das einen erwünschten osmotischen Druck geeignet für den Zustand des Patienten hat, und daß der Rechner (20) mit einer Anzeigeeinrichtung zum Anzeigen der berechneten eingespritzen und entnommenen Mengen verbunden ist.

2. Vorrichtung nach Anspruch 1, bei der der Fließmittelkreis einen Überführungsleitungsschalter (17) enthält, der aus einer Zumeßklemme (17A), einer Einspritzklemme (17B) und einer Entnahmeklemme (17C) besteht, die so zusammenarbeiten, daß der Fluß des Dialysats von der Speichereinheit (14) zu dem Patienten oder von dem Patienten zu der Entnahmeeinheit (15) umgeschaltet wird.

3. Vorrichtung nach einem der Ansprüche 1 und 2, bei der die Dialysatbeschickungseinheit und/oder die Dialysatspeichereinheit Beutel (11, 14) aufweisen, die zum Halten von Dialysat geeignet sind.

4. Vorrichtung nach einem der vorausgehenden Ansprüche, bei der die Heizeinrichtung einen Kunstharzbeutel (13A), der an einem mittleren Abschnitt des Fließmittelkreises vorgesehen ist, und Erhitzer (13B), zwischen denen der Beutel sandwichartig angeordnet ist und die das Dialysat durch Wärmeleitung erhitzen können, aufweist.

5. Vorrichtung nach einem der vorausgehenden Ansprüche, bei der die Heizeinrichtung (13) und die Dialysatspeichereinheit (14) durch eine einzige Überführungsleitungslänge direkt miteinander verbunden sind.

## Revendications

1. Appareil de dialyse péritonéale, comprenant une unité d'alimentation de solution de dialyse, laquelle comprend une pluralité de récipients à solution de dialyse (11), du même type, une unité de stockage de solution de dialyse (14) destinée à stocker provisoirement la solution de dialyse provenant de l'unité d'alimentation de solution de dialyse, un moyen (12) pour transférer la solution de dialyse provenant de l'unité d'alimentation à l'unité de stockage, un réchauffeur (13) destiné à chauffer la solution de dialyse avant qu'elle ne soit injectée dans le corps d'un patient, et une unité d'évacuation de solution de dialyse (15), destinée à recevoir la solution de dialyse après qu'elle a traversé le corps du patient, toutes les unités étant raccordées à un circuit de fluide, régulable, à l'aide d'un tube de transfert (16), le circuit de fluide étant régulé à l'aide de moyens de pesage (18A, 18B) pour l'unité de stockage de solution de dialyse (14) et l'unité d'évacuation de solution de dialyse (15), lesquels sont électriquement raccordés à une calculatrice (20), qui va calculer, à partir des deux valeurs numériques obtenues des moyens de pesage (18A, 18B), les quantités de solution de dialyse injectées dans le patient et évacuées du patient, chaque récipient à solution de dialyse (11) étant fixé d'une manière amovible au circuit de fluide, caractérisé en ce que chaque récipient à solution de dialyse (11) est raccordé en parallèle à un autre, et contient une solution de dialyse ayant une composition différente de celle de l'autre récipient, de telle sorte que la solution de dialyse de chaque récipient puisse être mélangée à la solution de dialyse provenant d'un autre récipient, au niveau de l'orifice d'entrée du tube de transfert (16), pour produire une solution de dialyse composite présentant une pression osmotique souhaitée, adaptée à l'état du patient, et en ce que la calculatrice (20) est raccordée à un écran, pour visualiser les quantités injectées et évacuées telles que calculées.

2. Appareil selon la revendication 1, dans lequel le circuit de fluide comprend un commutateur de tube de transfert (17), constitué d'un clamp de dosage (17A), d'un clamp d'injection (17B) et d'un clamp d'évacuation (17C), qui coopèrent pour commuter l'écoulement de la solution de dialyse, de l'unite de stockage (14) au patient, ou du patient à l'unité d'évacuation (15).

3. Appareil selon l'une quelconque des revendications 1 ou 2, dans lequel l'unité d'alimentation de solution de dialyse et/ou l'unité de stockage de solution de dialyse comprennent des poches (11, 14) pouvant contenir la solution de dialyse.

4. Appareil selon l'une quelconque des revendications précédentes, dans lequel l'unité de chauffage comprend une poche (13A) en résine synthétique, installée sur une portion intermédiaire du circuit de fluide, et des éléments chauffants (13B) entre lesquels est coincée la poche, et qui peuvent chauffer la solution de dialyse par conduction thermique.

5. Appareil selon l'une quelconque des revendications précédentes, dans lequel l'unité de chauffage (13) et l'unité de stockage de solution de dialyse (14) sont directement raccordées l'une à l'autre par un tronçon unique de tube de transfert.

FIG.1

FIG. 2

FIG. 3